# EUROPEAN PATENT APPLICATION

(11) **EP 4 494 648 A1**
(43) Date of publication of application: **22.01.2025**
(21) Application number: 23770929.0
(22) Date of filing: 17.03.2023
(51) Int. Cl.: A61K 36/53, A23B 7/02, A23B 7/06, A23L 19/00, A23L 33/105, A61K 8/9789, A61K 31/192, A61K 31/216, A61K 31/352, A61K 31/7048, A61K 36/534, A61K 36/535, A61P 13/00, A61P 13/02, A61P 27/14, A61P 29/00, A61P 37/00, A61P 37/08, A61Q 19/00, A61K 127/00

(54) **METHOD FOR MANUFACTURING LABIATAE HOT-WATER EXTRACT**

(30) Priority: 18.03.2022 JP 2022043375; 18.03.2022 JP 2022043376; 28.10.2022 JP 2022173485
(71) Applicant: Kao Corporation, Tokyo 103-8210 (JP)
(72) Inventor: SAITO, Keita, Haga-gun, Tochigi 321-3497 (JP); IWASHITA, Masazumi, Haga-gun, Tochigi 321-3497 (JP); HASHIZUME, Kohjiro, Haga-gun, Tochigi 321-3497 (JP); TAKAMURA, Akihiro, Haga-gun, Tochigi 321-3497 (JP); KAYAKUBO, Daisuke, Wakayama-shi, Wakayama 640-8580 (JP); DOI, Haruhiko, Wakayama-shi, Wakayama 640-8580 (JP)
(74) Representative: Vossius & Partner Patentanwälte Rechtsanwälte mbB
(86) International application number: PCT/JP2023/010751
(87) International publication number: WO 2023/176977

(57) **Abstract**

Provided is a method capable of industrially advantageously producing a Lamiaceae plant extract containing rosmarinic acid at a high concentration. A method for producing a hot-water extract of a Lamiaceae plant including the following steps (A) and (B): (A) subjecting a Lamiaceae plant to a blanching treatment at 85°C or higher, then drying; and (B) extracting a dried product obtained in the step (A) with hot water.

## Description

### Field of the Invention

The present invention relates to a method for producing a Lamiaceae plant hot-water extract and a dried product of a Lamiaceae plant.

### Background of the Invention

Lamiaceae plants have been used for edible and medicinal purposes since ancient times. A known medicinal component of the Lamiaceae plants is rosmarinic acid, which is mainly contained in the leaves. Rosmarinic acid has been reported to have anti-allergic effects, anti-inflammatory effects, and preventive or ameliorative effects on nocturia (Non Patent Literature 1, Patent Literature 1).

Examples of the methods reported for producing an extract containing rosmarinic acid from a Lamiaceae plant include a production method of a rosmarinic acid-containing Lamiaceae plant extract, in which whole fresh leaves of red perilla are extracted with an organic solvent, water or a mixture thereof under acidic conditions, and then purified by chromatography using an adsorbent resin (Patent Literature 2), and a production method of an extract solution having a high content of rosmarinic acid, in which perilla leaves are heat-treated with vapor, then lyophilized, and then extracted with ethanol (Patent Literature 3).
Patent Literature 1: JP-A-2019-206519
Patent Literature 2: WO 2002/62365
Patent Literature 3: JP-A-2013-51908
Non Patent Literature 1: Hirotsugu Kido, Oleo Science, 2004, 4(10), p. 409-415

### Summary of the Invention

The present invention relates to the following (1) to (3).
(1) A method for producing a hot-water extract of a Lamiaceae plant, the method comprising the following steps (A) and (B):
   (A) subjecting a Lamiaceae plant to a blanching treatment at 85°C or higher, then drying; and
   (B) extracting a dried product obtained in the step (A) with hot water.
(2) A hot-water extract of a Lamiaceae plant, having a mass ratio of a content of rosmarinic acid to a content of luteolin being 1,100 or more.
(3) A method for producing a dried product of a Lamiaceae plant, the method comprising the following step (A1) :
   (A') subjecting a Lamiaceae plant to a blanching treatment in the presence of an organic acid, then drying.

### Detailed Description of the Invention

The method of Patent Literature 2, however, has problems in terms of industrial productivity, such as the low purity of rosmarinic acid in the obtained extract itself and the required purification process to achieve high purity. Furthermore, Patent Literatures 2 and 3 use hydrous organic solvents for extraction, which require a removal process for the solvents after extraction, making the work processes complicated.

Thus, the present invention relates to a provision of a method capable of industrially advantageously producing a Lamiaceae plant extract containing rosmarinic acid at a high concentration.

The present inventors found that rosmarinic acid can be recovered from a Lamiaceae plant in a high yield by subjecting a Lamiaceae plant to a blanching treatment, then drying, and extracting the obtained dried product with hot water, and that a Lamiaceae plant extract containing rosmarinic acid at a high concentration can be obtained without a purification step.

The present inventors also found that the concentration of rosmarinic acid in the dried product after drying can be increased by subjecting a Lamiaceae plant to a blanching treatment in the presence of an organic acid, and further found that rosmarinic acid can be recovered in a high yield by extracting the dried product with hot water, so that a Lamiaceae plant extract containing rosmarinic acid at a high concentration can be obtained without a purification step.

According to the present invention, a Lamiaceae plant hot-water extract and a dried product of a Lamiaceae plant, which contain rosmarinic acid at high concentrations, can be obtained in a high yield.

### [Method for producing Lamiaceae plant hot-water extract]

The method for producing a Lamiaceae plant hot-water extract of the present invention includes a step (A) of subjecting a Lamiaceae plant to a blanching treatment at 85°C or higher, then drying; and a step (B) of extracting a dried product obtained in the step (A) with hot water.

### [Step (A)]

The step (A) of the present invention is a step of subjecting a Lamiaceae plant to a blanching treatment at 85°C or higher, then drying.

### (Lamiaceae plant)

The Lamiaceae plant in the present invention is not particularly limited as long as it is a plant classified as a family of Lamiaceae, and examples thereof include perilla (shiso), perilla sesame (egoma), rosemary, lavender, salvia, sage, mint, peppermint, spearmint, lemon balm, basil, and thyme. One or more Lamiaceae plants can be used. Among them, plants of genus Perilla such as red perilla (aka-shiso) and green perilla (aoshiso), plants of genus Salvia such as rosemary, and plants of genus Mentha such as peppermint and spearmint are preferred, and red perilla, green perilla, rosemary, and spearmint are more preferred, from the viewpoint of the extraction rate of rosmarinic acid. One or more of them can be used.

Used parts of the Lamiaceae plant are not particularly limited, and examples thereof include a whole body, a leaf, a stem, a bud, a flower, a branch, a root, a seed, and the like, and a mixture thereof. Among them, the leaf is preferred from the viewpoint of rosmarinic acid extraction rate.

The Lamiaceae plant used may be fresh, salted, or the like, but from the viewpoint of production cost, a fresh plant is preferably used.

### (Blanching treatment)

The blanching treatment is a processing method of heating a fruit or a vegetable and cooling it for a short time. The blanching treatment is performed for the purpose of preventing quality deterioration during processing and storage, and the like by heat-inactivating enzymes and sterilizing microorganisms. Examples of the blanching treatment include a steam blanching treatment, a boil blanching treatment, and a microwave irradiation treatment. These treatments can be used singly or in combination.

The steam blanching treatment may include bringing a Lamiaceae plant into contact with, for example, water vapor, and heating.

The temperature of the water vapor is preferably 85°C or higher, more preferably 87°C or higher, and even more preferably 90°C or higher from the viewpoint of heat inactivation for polyphenol oxidase. The upper limit of the temperature is not particularly limited, but is preferably 100°C or lower.

The boil blanching treatment may include immersing a Lamiaceae plant in, for example, hot boiled water, and heating.

The temperature of the hot boiled water is preferably 85°C or higher, more preferably 87°C or higher, and even more preferably 90°C or higher from the viewpoint of heat inactivation for polyphenol oxidase. The upper limit of the temperature is not particularly limited, but is preferably 100°C or lower.

The microwave irradiation treatment may include irradiating a Lamiaceae plant with microwave, for example, at a frequency of 2,450 MHz.

In this step, the steam blanching treatment and boil blanching treatment are preferred, and boil blanching treatment is more preferred, from the viewpoint of heat inactivation for polyphenol oxidase and production cost.

In this step, it is also preferable to perform the blanching treatment in the presence of common salt. The blanching treatment in the presence of common salt can increase the concentration of rosmarinic acid in the dried product of a Lamiaceae plant provided to a next step.

The common salt may be present at the time of the blanching treatment. For example, in the case of a steam blanching treatment, the Lamiaceae plant may be mixed with common salt, and then the mixture may be brought into contact with water vapor, and heated.

In the case of a boil blanching treatment, the mixing order of the Lamiaceae plant, common salt, and hot boiled water is not particularly limited, and they can be mixed in any order. The Lamiaceae plant and common salt may be mixed and then immersed in hot boiled water, or the Lamiaceae plant may be immersed in hot boiled water that has been mixed with common salt.

When performing the boil blanching treatment in the presence of common salt, a concentration of common salt in the hot boiled water is preferably 0.1 mass% or more and 30 mass% or less, more preferably 1 mass% or more and 15 mass% or less, and further preferably 5 mass% or more and 10 mass% or less from the viewpoint of suppressing rosmarinic acid elution.

In this step, it is also preferable to perform the blanching treatment in the presence of an organic acid. The blanching treatment in the presence of an organic acid can increase the concentration of rosmarinic acid in the dried product of a Lamiaceae plant provided to a next step.

The organic acid used in the present invention is not particularly limited, but preferably one or more selected from the group consisting of hydroxycarboxylic acids, dicarboxylic acids, and ascorbic acids.

Examples of the hydroxycarboxylic acids include lactic acid, citric acid, isocitric acid, malic acid, tartaric acid, gluconic acid, and quinic acid.

Examples of the dicarboxylic acids include oxalic acid, malonic acid, succinic acid, glutaric acid, adipic acid, fumaric acid, and maleic acid.

Examples of the ascorbic acids include L-ascorbic acid and erythorbic acid, which are stereoisomers.

Among them, the organic acid is preferably a hydroxycarboxylic acid or an ascorbic acid, more preferably citric acid or L-ascorbic acid from the viewpoint of increasing the concentration of rosmarinic acid in the dried product.

The organic acid may be present at the time of the blanching treatment. For example, in the case of a steam blanching treatment, the Lamiaceae plant may be mixed with an organic acid, and then the mixture may be brought into contact with water vapor, and heated.

In the case of a boil blanching treatment, the mixing order of the Lamiaceae plant, an organic acid, and hot boiled water is not particularly limited, and they can be mixed in any order. The Lamiaceae plant and an organic acid may be mixed and then immersed in hot boiled water, or the Lamiaceae plant may be immersed in hot boiled water that has been mixed with an organic acid.

When performing the boil blanching treatment in the presence of an organic acid, a concentration of the organic acid in the hot boiled water is preferably 0.1 mass% or more and 30 mass% or less, more preferably 0.5 mass% or more and 15 mass% or less, and even more preferably 1 mass% or more and 10 mass% or less from the viewpoint of suppressing rosmarinic acid elution.

The time of the blanching treatment can be appropriately set depending on the type and used part of the Lamiaceae plant, but preferably 15 seconds or more and 15 minutes or less, more preferably 30 seconds or more and 10 minutes or less, further preferably 60 seconds or more and 5 minutes or less from the viewpoint of heat inactivation for polyphenol oxidase and suppression of rosmarinic acid elution. After the blanching treatment, the Lamiaceae plant may be air-cooled or water-cooled, or subjected to draining of water before drying.

### (Drying)

As the method for drying, known methods such as spray drying, hot air drying, lyophilization, or vacuum (decompression) drying can be employed. Among them, hot air drying is preferred from the viewpoint of production cost.

The drying temperature is preferably 50°C or higher from the viewpoint of drying rate and growth inhibition of microorganisms.

The drying time can be adjusted as appropriate, but preferably 3 hours or more and 12 hours or less from the viewpoint of moisture removal.

### [Step (B)]

The step (B) of the present invention is a step of extracting the dried product obtained in the step (A) with hot water.

### (Extraction means)

In the extracting, any means such as immersion, decoction, leaching, reflux extraction, ultrasonic extraction, and the like can be used. The extraction may be performed once or repeated plural times (e.g., twice, or three times).

The temperature of hot water used for the extracting is preferably 40°C or higher, more preferably 70°C or higher, and further preferably 90°C or higher from the viewpoint of the extraction rate of rosmarinic acid and the growth inhibition of microorganisms. The upper limit of the temperature of hot water is not particularly limited, but is preferably 100°C or lower.

Examples of the type of water include tap water, distilled water, ion exchange water, and purified water.

During the extraction of the dried product, pH adjustment by adding an agent is not required. When pH adjustment is performed, the pH is 5 or more and less than 9 (20°C), more preferably 6 or more and less than 8.

In the step (A), when the boil blanching treatment is performed in the presence of an organic acid, pH at the time of extraction (20°C) is preferably 4 or less from the viewpoint of suppressing rosmarinic acid elution.

The extraction time is preferably 30 minutes or more, more preferably 1 hour or more from the viewpoint of the extraction rate of rosmarinic acid.

The bath ratio of hot water, that is, the mass ratio of hot water/dried product of Lamiaceae plant is preferably 10 or more and less than 1,000, more preferably 30 or more and less than 300 from the viewpoint of reducing the extraction solvent.

After extraction, the dried product of the Lamiaceae plant may be separated from the Lamiaceae plant hot-water extract by known solid-liquid separation means such as filtration, centrifugation, or membrane treatment.

In the present invention, the solid yield is preferably 10% or more, more preferably 12% or more.

In step (A), when the boil blanching treatment is performed in the presence of an organic acid, the solid yield is preferably 16% or more, more preferably 18% or more.

### [Method for producing dried product of Lamiaceae plant]

The method for producing a dried product of a Lamiaceae plant of the present invention includes a step (A') of subjecting a Lamiaceae plant to a blanching treatment in the presence of an organic acid, then drying.

### [Step (A')]

The step (A') of the present invention is a step of subjecting a Lamiaceae plant to a blanching treatment in the presence of an organic acid, then drying.

The specific compositions of the Lamiaceae plant, organic acid, blanching treatment, and drying are as described above.

The dried product of the Lamiaceae plant obtained by the step (A') can be used as is. The dried product of the Lamiaceae plant can also be subjected to the above-described step (B) to produce a hot-water extract of the Lamiaceae plant.

In the dried product of the Lamiaceae plant of the present invention, a content of rosmarinic acid is preferably 1 mass% or more, more preferably 2 mass% or more, and further preferably 3 mass% or more.

### [Lamiaceae plant hot-water extract]

The obtained Lamiaceae plant hot-water extract of the present invention may be used as is, or may be concentrated or dried.

Examples of concentration method include a normal pressure concentration method in which the solvent is evaporated at normal pressure, a decompression concentration method in which the solvent is evaporated under reduced pressure, and a membrane concentration method in which the solvent is removed by membrane separation.

Examples of drying method include known methods as described above. Among them, from the viewpoint of cost reduction and productivity improvement, the spray drying method and the lyophilization method are preferred.

Examples of form of the Lamiaceae plant hot-water extract include a liquid, a slurry, a semi-solid, and a solid.

The content of rosmarinic acid in the solids in the Lamiaceae plant hot-water extract of the present invention is preferably 4 mass% or more, more preferably 5 mass% or more, and further preferably 10 mass% or more. The term "solid(s)" as used herein refers to a residue obtained by concentrating a sample under reduced pressure with a rotary evaporator, followed by addition of water, lyophilization, and removal of volatiles.

The amount of luteolin in the Lamiaceae plant hot-water extract of the present invention is reduced by undergoing the above steps (A) and (B). Furthermore, the amount of luteolin-O-7-diglucuronide is increased in the Lamiaceae plant hot-water extract, such as the red perilla hot-water extract. Luteolin-O-7-diglucuronide and luteolin are components contained in the Lamiaceae plant. Luteolin-O-7-diglucuronide has been found to have health functions such as antioxidation-radical scavenging, antioxidation-active oxygen scavenging, binding inhibition against the central nervous system inhibitory receptors (Hennebelle, T. et al. Phytother. Res. 2008, 22. 256-258), amelioration of eye fatigue (WO 2020/017674), NO scavenging activity (Fraisse, D. et al. Molecules 2018, 23(7), 1574), and amelioration of isoproterenol-induced myocardial injury and fibrosis in mice (Ning, B. et al. Acta Pharmacologica Sinica 2017, 38, 331-341). The Lamiaceae plant hot-water extract of the present invention can thus be expected to have high functional expressions of rosmarinic acid and luteolin-O-7-diglucuronide.

The content of luteolin-O-7-diglucuronide in the solids is preferably 2 mass% or more in the Lamiaceae plant hot-water extract, such as the red perilla hot-water extract, of the present invention.

The mass ratio of a content of rosmarinic acid to a content of luteolin-O-7-diglucuronide [content of rosmarinic acid in solid/content of luteolin-O-7-diglucuronide in solid] in the Lamiaceae plant hot-water extract, such as the red perilla hot-water extract, is preferably from 0.9 to 12, more preferably from 4 to 8.

When the amount of luteolin is high, turbidity can be observed in an aqueous solution of the Lamiaceae plant hot-water extract containing high concentrations of rosmarinic acid. However, the amount of luteolin is small in the Lamiaceae plant hot-water extract of the present invention, thus the aqueous solution of the Lamiaceae plant hot-water extract of the present invention is highly transparent.

The content of luteolin in solids in the Lamiaceae plant hot-water extract of the present invention is preferably 100 ppm or less.

The mass ratio of a content of rosmarinic acid to a content of luteolin [content of rosmarinic acid in solid/content of luteolin in solid] in the Lamiaceae plant hot-water extract is preferably 1,100 or more, more preferably 2,500 or more, and further preferably 5,000 or more.

The Lamiaceae plant hot-water extract of the present invention can be used in various food and beverages, pharmaceuticals, quasi-drugs, cosmetics, and the like.

Regarding the above-described embodiments, the following aspects are further disclosed in the present invention.
<1> A method for producing a hot-water extract of a Lamiaceae plant, the method comprising the following steps (A) and (B):
   (A) subjecting a Lamiaceae plant to a blanching treatment at 85°C or higher, then drying; and
   (B) extracting a dried product obtained in the step (A) with hot water.
<2> The production method according to <1>, wherein the Lamiaceae plant is preferably a plant of genus Perilla of family Lamiaceae, more preferably red perilla.
<3> The production method according to <1> or <2>, wherein the blanching treatment is preferably a steam blanching treatment or a boil blanching treatment, more preferably a boil blanching treatment.
<4> The production method according to <3>, wherein a temperature of water vapor or a temperature of hot boiled water is preferably 85°C or higher, more preferably 87°C or higher, further preferably 90°C or higher, and preferably 100°C or lower.
<5> The production method according to any one of <1> to <4>, wherein the blanching treatment is preferably performed in the presence of common salt.
<6> The production method according to <5>, wherein a concentration of common salt in hot boiled water when performing the boil blanching treatment is preferably 0.1 mass% or more and 30 mass% or less, more preferably 1 mass% or more and 15 mass% or less, further preferably 5 mass% or more and 10 mass% or less.
<7> The production method according to any one of <1> to <4>, wherein the blanching treatment is preferably performed in the presence of an organic acid.
<8> The production method according to <7>, wherein the organic acid is preferably one or more selected from the group consisting of hydroxycarboxylic acids, dicarboxylic acids and ascorbic acids, more preferably one or more selected from the group consisting of hydroxycarboxylic acids and ascorbic acids, and further preferably citric acid, L-ascorbic acid or a combination thereof.
<9> The production method according to <7> or <8>, wherein a concentration of the organic acid in hot boiled water when performing the boil blanching treatment is preferably 0.1 mass% or more and 30 mass% or less, more preferably 0.5 mass% or more and 15 mass% or less, further preferably 1 mass% or more and 10 mass% or less.
<10> The production method according to any one of <1> to <9>, wherein a time of the blanching treatment is preferably 15 seconds or more and 15 minutes or less, more preferably 30 seconds or more and 10 minutes or less, further preferably 60 seconds or more and 5 minutes or less.
<11> The production method according to any one of <1> to <10>, wherein a method for the drying is preferably spray drying, hot air drying, lyophilization, or vacuum (decompression) drying, more preferably hot air drying.
<12> The production method according to any one of <1> to <11>, wherein a temperature of the hot water used for the extracting is preferably 40°C or higher, more preferably 70°C or higher, even more preferably 90°C or higher, and preferably 100°C or lower.
<13> The production method according to any one of <1> to <12>, wherein a bath ratio of the hot water is preferably 10 or more and less than 1,000, more preferably 30 or more and less than 300.
<14> A Lamiaceae plant hot-water extract, having a mass ratio of a content of rosmarinic acid to a content of luteolin being 1,100 or more.
<15> The Lamiaceae plant hot-water extract according to <14>, wherein the mass ratio of a content of rosmarinic acid to a content of luteolin is preferably 2,500 or more, more preferably 5,000 or more.
<16> A method for producing a dried product of a Lamiaceae plant, the method comprising a step (A') of subjecting a Lamiaceae plant to a blanching treatment in the presence of an organic acid, then drying.
<17> The production method according to <16>, wherein the organic acid is preferably one or more selected from the group consisting of hydroxycarboxylic acids, dicarboxylic acids, and ascorbic acids, more preferably one or more selected from the group consisting of hydroxycarboxylic acids and ascorbic acids, and further preferably citric acid, L-ascorbic acid or a combination thereof.

### Examples

### [Quantification of rosmarinic acid and luteolin-O-7-diglucuronide, LC-UV]

The contents of rosmarinic acid and luteolin-O-7-diglucuronide were quantified under the following conditions.
HPLC: Agilent 1260 Infinity
Column: CAPCELL CORE C18 (2.1 mml × 100 mm 2.7 µm) Product number 51105 (OSAKA SODA CO., LTD.)
Eluent: A 0.1 vol% aqueous formic acid solution, B acetonitrile
Flow rate: 0.7 mL/min
Temperature: 40°C
Detection wavelength: rosmarinic acid 320 nm, luteolin-O-7-diglucuronide 350 nm
Inject: solid content 1.00 mg/mL, 5.0 µL
Calibration curve

### - Rosmarinic acid

A calibration curve of rosmarinic acid was generated with 8 points between 1,000 to 10 µg/mL prepared using a commercially available reagent (Carbosynth Limited, 98.2% purity) diluted with 50 vol% aqueous ethanol solution.

### - Luteolin-O-7-diglucuronide

A calibration curve of luteolin-O-7-diglucuronide was generated with 8 points between 1,000 to 10 µg/mL prepared using a commercially available reagent (Sigma-Aldrich Co. LLC, 98.2% purity) diluted with 50 vol% aqueous ethanol solution.

### Quantification sample

Powder: 10.0 mg of an extract powder was dissolved in 10.0 mL of ion exchange water to afford a 1.00 mg/mL solution, which was analyzed by HPLC-UV.

Extract solution: An extraction solution of dried leaves of a Lamiaceae plant was diluted 10-fold with ion exchange water and analyzed by HPLC-UV.

**[Table 1]**

| Elution conditions | | | | | |
|---|---|---|---|---|---|
| time (min) | 0-0.6 | 2.0-4.4 | 5.4-9 | 9.1-11 | 11.1-15 |
| Eluent vol%A | 94 | 82 | 75 | 3 | 94 |
| Eluent vol%B | 6 | 18 | 25 | 97 | 6 |

### [Quantification of luteolin, LC-MS/MS]

The content of luteolin was measured under the following conditions.
HPLC: Sciex ExionLC (AB Sciex Pte. Ltd.)
MS/MS: TRIPLE QUAD 4500 (AB Sciex Pte. Ltd.)
Column: Capcell Core C18 (2.1 mm × 50 mm, 2.7 µm) Product number 51103 (OSAKA SODA CO., LTD.)
Eluent: A 0.1 vol% aqueous formic acid solution, B acetonitrile
Flow rate: 0.7 mL/min
Temperature: 40°C
Inject: 50 vol% aqueous ethanol solution

### Calibration curve:

A calibration curve thereof was generated with 12 points between 1,000 to 0.25 µg/mL prepared using a commercially available reagent luteolin (Fujifilm Wako Pure Chemical Corporation, 95% purity) diluted with 50% aqueous ethanol solution.

**[Table 2]**

| Elution conditions | | | | | |
|---|---|---|---|---|---|
| time (min) | 0 | 4.0 | 4.5 | 4.51 | 5.0 |
| Eluent vol%A | 80 | 30 | 30 | 80 | 80 |
| Eluent vol%B | 20 | 70 | 70 | 20 | 20 |

| MS/MS detection conditions | | | | | |
|---|---|---|---|---|---|
| | Q1 | Q3 | DP(V) | CE(V) | CXP(V) |
| Luteolin | 286.784 | 153.000 | 121 | 45 | 8 |

### [Red perilla hot-water extract]

### Examples 1-10

100 g of red perilla fresh leaves (katamen-shiso grown in China) were subjected to a boil blanching treatment (90°C, 1 minute) with 2 L of hot boiled water, then cooled with water of room temperature, and further subjected to hot air drying (60°C, 12 hours) to afford 13.5 g of boiled dried leaves.

Then, under the conditions shown in Table 3, the boiled dried leaves were extracted with hot water, filtered with a 0.45 µm PES filter, and then lyophilized to afford an extract powder of red perilla hot-water extract.

The rosmarinic acid content (mass%), luteolin-7-O-diglucuronide content (mass%), and luteolin content (mass ppm) in the obtained red perilla extract powder were quantified.

The treatment conditions and results are shown in Table 3. In Table 3, rosmarinic acid was abbreviated as RA, luteolin-7-O-diglucuronide was abbreviated as L7DG, and luteolin was abbreviated as Lut.

The solid recovery rate was calculated by the following equation (1). Solid recovery rate (%) = weight of extract powder/weight of extraction raw material dried leaves × 100 (%)

**[Table 3]**

| Example | Boiling conditions | | | Hot-water extraction conditions | | | Hot-water extract yield | | Composition | | | Component amount ratio | |
|---|---|---|---|---|---|---|---|---|---|---|---|---|---|
| | Raw material leaves | Temperature °C | Time min | Temperat ure °C | Time min | Bath ratio fold | Solid yield % | RA recovered amount mg/1 g processed leaves | RA mass% | L7DG mass% | Lut mass ppm | RA/L7DG | RA/Lut |
| Example 1 | | | | 90 | 360 | 50 | 15.2 | 19.6 | 12.9 | 2.2 | 1.5 | 6.0 | 86313 |
| Example 2 | | | | 90 | 180 | | 14.8 | 21.9 | 14.8 | 2.6 | 4.1 | 5.8 | 36000 |
| Example 3 | | | | 90 | 60 | | 14.3 | 23.2 | 16.3 | 2.3 | 4.4 | 7.1 | 36987 |
| Example 4 | | | | 90 | 20 | | 14.1 | 24.5 | 17.4 | 2.6 | 2.7 | 6.6 | 64293 |
| Example 5 | Katamen-shiso grown in China | 90 | 1 | 70 | 90 | | 14.6 | 22.3 | 15.3 | 2.5 | <0.1 | 6.1 | >1530732 |
| Example 6 | | | | 50 | 180 | | 14.8 | 21.8 | 14.8 | 2.6 | <0.1 | 5.6 | >1478070 |
| Example 7 | | | | 50 | 60 | | 14.5 | 24.6 | 17.0 | 2.7 | <0.1 | 6.3 | >1699497 |
| Example 8 | | | | 90 | 60 | 33 | 17.4 | 27.1 | 15.6 | 3.8 | 0.6 | 6.4 | 250867 |
| Example 9 | | | | 90 | 15 min*3 times | Total 50 | 17.3 | 28.2 | 16.3 | 3.9 | 0.9 | 6.5 | 186967 |
| Example 10 | | | | 90 | 15 min*6 times | Total 100 | 19.4 | 33.2 | 17.1 | 4.1 | 1.4 | 7.2 | 121155 |

### Examples 11-16, Comparative Examples 1-4

100 g of red perilla fresh leaves (chirimen-aka-shiso grown in Hiroshima prefecture) were subjected to a boil blanching treatment with 2 L of hot boiled water under the conditions shown in Table 4, then cooled with water of room temperature, and further subjected to hot air drying (60°C, 12 hours) to afford 13.5 g of boiled dried leaves.

Then, the boiled dried leaves were extracted with hot water (90°C, 60 min, bath ratio of 50-fold), filtered with a 0.45 µm PES filter, and then lyophilized to afford an extract powder of red perilla hot-water extract.

The rosmarinic acid content (mass%), luteolin-7-O-diglucuronide content (mass%), and luteolin content (mass ppm) in the obtained red perilla extract powder were quantified.

The treatment conditions and results are shown in Table 4.

**[Table 4]**

| Example | Boiling conditions | | | Hot-water extraction conditions | | | Hot-water extract yield | | Composition | | | Component amount ratio | |
|---|---|---|---|---|---|---|---|---|---|---|---|---|---|
| | Raw material leaves | Temperature °C | Time min | Temperat ure °C | Time min | Bath ratio fold | Solid yield % | RA recovered amount mg/1 g leaves | RA mass% | L7DG mass% | Lut mass ppm | RA/L7DG | RA/Lut |
| Example 11 | | 90 | 0.5 | | | | 11.4 | 15.2 | 13.3 | 5.9 | 20.9 | 2.2 | 6342 |
| Example 12 | | 90 | 1 | | | | 12.0 | 18.1 | 15.1 | 5.7 | 13.4 | 2.7 | 11294 |
| Example 13 | | 90 | 5 | | | | 9.8 | 14.9 | 15.2 | 4.8 | 11.4 | 3.2 | 13306 |
| Example 14 | | 90 | 10 | | | | 9.8 | 14.3 | 14.6 | 5.5 | 20.4 | 2.7 | 7159 |
| Example 15 | Chirimen-aka-shiso grown in Hiroshima prefecture | 90 | 15 | 90 | 60 | 50 | 5.2 | 6.0 | 11.5 | 4.6 | 16.7 | 2.5 | 6869 |
| Example 16 | | 85 | 1 | | | | 14.3 | 6.1 | 4.3 | 4.6 | 14.0 | 0.9 | 3044 |
| Comparative Example 1 | | 80 | 1 | | | | 13.5 | 0.5 | 0.4 | 2.9 | 6.1 | 0.1 | 627 |
| Comparative Example 2 | | 75 | 1 | | | | 15.7 | 0.3 | 0.2 | 1.0 | 2.2 | 0.2 | 1046 |
| Comparative Example 3 | | 70 | 1 | | | | 14.3 | 0.4 | 0.3 | 1.1 | 4.4 | 0.3 | 698 |
| Comparative Example 4 | | 60 | 1 | | | | 18.1 | 0.4 | 0.3 | 3.4 | 18.6 | 0.1 | 140 |

### Example 17

100 g of red perilla fresh leaves (chirimen-aka-shiso grown in Hiroshima prefecture) were subjected to a boil blanching treatment (90°C, 1 minute) with 2 L of hot boiled water, then cooled with water of room temperature, and further subjected to hot air drying (60°C, 12 hours) to afford 8.8 g of boiled dried leaves.

Then, 600 mg of the dried leaves were extracted with hot water (90°C, 60 min, bath ratio of 50-fold), filtered with a 0.45 µm PES filter, and then lyophilized to afford 86.4 mg of an extract powder of red perilla hot-water extract (solid yield of 14.4%). The rosmarinic acid content in the obtained red perilla extract powder was 4.7 mass%.

### Examples 18-20 (Boil in aqueous common salt solution)

100 g of chirimen-aka-shiso fresh leaves grown in Hiroshima prefecture as in Example 17 were subjected to a boil blanching treatment (90°C, 1 minute) with 2 L of 5 mass%, 1 mass%, or 0.1 mass% aqueous common salt solution, then cooled with water of room temperature, and further subjected to hot air drying (60°C, 12 hours) to afford 10.3 g of boiled dried leaves.

Then, 600 mg of the dried leaves were extracted with hot water (90°C, 60 min, bath ratio of 50-fold), filtered with a 0.45 µm PES filter, and then lyophilized to afford an extract powder of red perilla hot-water extract. The rosmarinic acid content (mass%) in the obtained red perilla extract powder was quantified.

The treatment conditions and results are shown in Table 5.

**[Table 5]**

| | Boil blanching conditions | | | | Hot-water extraction conditions | | | Hot-water extract yield | | Composition |
|---|---|---|---|---|---|---|---|---|---|---|
| | Raw material leaves | Boiling water | Temperature °C | Time min | Temperature °C | Time min | Bath ratio fold | Solid yield % | RA recovered amount mg/1 g leaves | RA mass% |
| Example 17 | Chirimen-aka-shiso grown in Hiroshima prefecture | Tap water | 90 | 1 | 90 | 60 | 50 | 14.4 | 6.8 | 4.7 |
| Example 18 | | 5 mass% aqueous common salt solution | | | | | | 24.4 | 33.9 | 13.9 |
| Example 19 | | 1 mass% aqueous common salt solution | | | | | | 23.4 | 25.3 | 10.8 |
| Example 20 | | 0.1 mass% aqueous common salt solution | | | | | | 20.7 | 24.0 | 11.6 |

### Example 21 (Steam Branching)

50.4 g of red perilla fresh leaves (katamen-aka-shiso grown in Kumamoto prefecture) were subjected to a steam treatment (98°C, 3 minutes) with boiling water, then cooled with water of room temperature, and further subjected to hot air drying (60°C, 12 hours) to afford 6.4 g of steamed dried leaves.

Then, 300 mg of the dried leaves were extracted with hot water (90°C, 60 min, bath ratio of 50-fold), filtered with a 0.45 µm PES filter, and then lyophilized to afford 55.2 mg of an extract powder of red perilla hot-water extract (solid yield of 18.4%). The rosmarinic acid content in the obtained red perilla extract powder was 8.7 mass%.

### Example 22 (Mixing with common salt)

50.2 g of katamen-aka-shiso fresh leaves grown in Kumamoto prefecture as in Example 21 were mixed with 50.0 g of common salt, and immersed for 10 minutes. The mixture was subjected to a boil blanching treatment (90°C, 1 minute) with 1 L of hot boiled water, then cooled with water of room temperature, and further subjected to hot air drying (60°C, 12 hours) to afford 7.2 g of boiled dried leaves.

Then, 300 mg of the dried leaves were extracted with hot water (90°C, 60 min, bath ratio of 50-fold), filtered with a 0.45 µm PES filter, and then lyophilized to afford 105 mg of an extract powder of red perilla hot-water extract (solid yield of 35.0%). The rosmarinic acid content in the obtained red perilla extract powder was 5.3 mass%.

### Example 23

50.4 g of katamen-aka-shiso fresh leaves grown in Kumamoto prefecture as in Example 21 were subjected to a boil blanching treatment (90°C, 1 minute) with 1 L of hot boiled water, then cooled with water of room temperature, and further subjected to hot air drying (60°C, 12 hours) to afford 6.1 g of boiled dried leaves.

Then, 300 mg of the dried leaves were extracted with hot water (90°C, 60 min, bath ratio of 50-fold), filtered with a 0.45 µm PES filter, and then lyophilized to afford 43.5 mg of an extract powder of red perilla hot-water extract (solid yield of 14.5%). The rosmarinic acid content in the obtained red perilla extract powder was 2.5 mass%.

The treatment conditions and results are shown in Table 6.

**[Table 6]**

| | Blanching conditions | | | | Hot-water extraction conditions | | | Hot-water extract yield | | Composition |
|---|---|---|---|---|---|---|---|---|---|---|
| | Raw material leaves | Treatment method | Temperature °C | Time min | Temperature °C | Time min | Bath ratio fold | Solid yield % | RA recovered amount mg/1 g leaves | RA mass% |
| Example 21 | Katamen-aka-shiso grown in Kumamoto prefecture | Steaminq | 98 | 3 | 90 | 60 | 50 | 18.4 | 16.0 | 8.7 |
| Example 22 | | Mixing with common salt, then boiling with tap water | 90 | 1 | | | | 35.0 | 18.5 | 5.3 |
| Example 23 | | Boiling with tap water | 90 | 1 | | | | 14.5 | 3.6 | 2.5 |

### Example 24 and Reference Examples 1 and 2

By the method of Example 3, 300 mg of boiled dried leaves of katamen-aka-shiso grown in China were extracted with 15 mL of hot water, 50 vol% hydrous ethanol, 80 vol% hydrous ethanol (90°C, 60 min, bath ratio of 50-fold), and an extract powder of the extract was prepared by ethanol removal by decompression concentration and lyophilization. The rosmarinic acid content in the extract solution and extract powder was quantified using HPLC-UV, and the rosmarinic acid recovered amount was quantified for each extraction solvent.

As a result, in the hot water extraction, the solid yield was 11.2%, the rosmarinic acid content was 17.8 mass%, and RA was 20.0 mg/1 g dried leaves (Example 24). In the extraction with 50 vol% hydrous ethanol, the solid yield was 12.2%, the rosmarinic acid content was 18.2 mass%, and RA was 22.2 mg/1 g dried leaves (Reference Example 1). In the extraction with 80 vol% hydrous ethanol, the solid yield was 11.9%, the rosmarinic acid content was 17.7 mass%, and RA was 21.0 mg/1 g dried leaves (Reference Example 2). In other words, the hot water extraction on the boil-blanched dried leaves gave an extract powder having about the same rosmarinic acid purity in about the same yield as the ethanol extraction.

### Examples 25-30

100 g of chirimen-aka-shiso fresh leaves grown in Hiroshima prefecture were subjected to a boil blanching treatment (90°C, 1 minute) with 2 L of 5.0 mass%, 1.0 mass%, or 0.1 mass% aqueous solution of citric acid or ascorbic acid, then cooled with water of room temperature, and further subjected to hot air drying (60°C, 12 hours) to afford organic acid-added boiled dried leaves.

The rosmarinic acid (RA) content in the dried leaves was measured by HPLC-UV quantification.

300 mg of the organic acid-added boiled dried leaves were extracted with hot water (90°C, 60 min, bath ratio of 50-fold), filtered with a 0.45 µm PES filter, and then lyophilized to afford a red perilla hot-water extract powder.

The rosmarinic acid content in the hot-water extract powder was measured by HPLC-UV quantification.

The solid recovery rate was calculated by the following equation (1). Solid recovery rate (%) = weight of extract powder/weight of extraction raw material dried leaves × 100 (%)

### Comparative Example 5

100 g of chirimen-aka-shiso fresh leaves grown in Hiroshima prefecture were subjected to a boil blanching treatment (90°C, 1 minute) with 2 L of hot boiled water, then cooled with water of room temperature, and further subjected to hot air drying (60°C, 12 hours) to afford boiled dried leaves.

The RA content in the dried leaves was measured by HPLC-UV quantification.

Boiled dried leaves were extracted with hot water (90°C, 60 min, bath ratio of 50-fold) as in Example 1, filtered with a 0.45 µm PES filter, and then lyophilized to afford a red perilla hot-water extract powder.

The rosmarinic acid content in the hot-water extract powder was measured by HPLC-UV quantification.

The treatment conditions and results are shown in Table 7.

**[Table 7]**

| | Boiling and drying conditions | | | | | | Hot-water extract | | Hot-water extract composition | | | Component amount ratio | |
|---|---|---|---|---|---|---|---|---|---|---|---|---|---|
| | Raw material leaves | Organic acid | Adding method | Boiling water mass% aqueous solution | Dry weight ratio (ratio to fresh leaves) | RA content in dried leaves mass% | Solid yield % | RA recovered amount mg/1 g dried leaves | RA % | L7DG % | Lut ppm | RAIL7DG | RAILut |
| Example 25 | Chirimen-aka-shiso grown in Hiroshima prefecture | Citric acid | Boil treatment with aqueous solution | 5.0 | 0.093 | 4.4 | 22.4 | 33.6 | 15.0 | 3.8 | 63.9 | 3.9 | 2347 |
| Example 26 | | | | 1.0 | 0.087 | 4.0 | 19.8 | 29.9 | 15.1 | 5.7 | 39.5 | 2.6 | 3818 |
| Example 27 | | | | 0.1 | 0.088 | 3.7 | 18.1 | 27.5 | 15.2 | 5.8 | 19.4 | 2.6 | 7844 |
| Example 28 | | Ascorbic acid | Boil treatment with aqueous solution | 5.0 | 0.093 | 4.9 | 22.5 | 35.3 | 15.7 | 4.8 | 98.2 | 3.2 | 1598 |
| Example 29 | | | | 1.0 | 0.091 | 4.6 | 20.8 | 34.7 | 16.7 | 5.8 | 37.9 | 2.9 | 4405 |
| Example 30 | | | | 0.1 | 0.088 | 3.6 | 20.2 | 27.7 | 13.7 | 5.4 | 17.7 | 2.5 | 7758 |
| Comparative Example 5 | | None | Boil treatment | - | 0.088 | 2.3 | 15.9 | 16.1 | 10.1 | 4.7 | 10.0 | 2.2 | 10100 |

### Examples 31-33

50 g of katamen-aka-shiso fresh leaves grown in Kumamoto prefecture were mixed with 50 g, 10 g, and 1.0 g of citric acid each, and immersed for 10 minutes, then subjected to a boil blanching treatment (90°C, 1 minute, final concentration of citric acid of 5 mass%, 1 mass%, or 0.1 mass%) with 1 L of hot boiled water, and then cooled with water of room temperature and subjected to hot air drying (60°C, 12 hours) to afford organic acid-added boiled dried leaves.

The RA content in the dried leaves was measured by HPLC-UV quantification.

300 mg of the organic acid-added boiled dried leaves were extracted with hot water (90°C, 60 min, bath ratio of 50-fold), filtered with a 0.45 µm PES filter, and then lyophilized to afford a red perilla hot-water extract powder.

The rosmarinic acid content in the hot-water extract powder was measured by HPLC-UV quantification.

### Comparative Example 6

50 g of katamen-aka-shiso fresh leaves grown in Kumamoto prefecture were subjected to a boil blanching treatment (90°C, 1 minute) with 1 L of hot boiled water, then cooled with water of room temperature, and further subjected to hot air drying (60°C, 12 hours) to afford boiled dried leaves.

The RA content in the dried leaves was measured by HPLC-UV quantification.

The boiled dried leaves were extracted with hot water (90°C, 60 min, bath ratio of 50-fold) as in Example 31, filtered with a 0.45 µm PES filter, and then lyophilized to afford a red perilla hot-water extract powder.

The rosmarinic acid content in the hot-water extract powder was measured by HPLC-UV quantification.

The treatment conditions and results are shown in Table 8.

**[Table 8]**

| | Boiling and drying conditions | | | | | | Hot-water extract | | Hot-water extract composition |
|---|---|---|---|---|---|---|---|---|---|
| | Raw material leaves | Organic acid | Adding method | Boiling water mass% aqueous solution | Dry weight ratio (ratio to fresh leaves) | RA content in dried leaves mass% | Solid yield % | RA recovered amount mg/1 g dried leaves | RA% |
| Example 31 | Katamen-aka-shiso grown in Kumamoto prefecture | Citric acid | Mixing fresh leaves with citric acid in advance and, then boil treatment with hot boiling water | 5.0 | 0.130 | 1.2 | 22.8 | 10.0 | 4.4 |
| Example 32 | | | | 1.0 | 0.109 | 1.6 | 16.5 | 10.4 | 6.3 |
| Example 33 | | | | 0.1 | 0.116 | 1.3 | 17.4 | 15.9 | 9.1 |
| Comparative Example 6 | | None | Boil treatment | 5.0 | 0.122 | 0.8 | 14.5 | 3.6 | 2.5 |

### [Hot-water extracts of green perilla, spearmint, and rosemary]

### Example 34

50.5 g of green perilla fresh leaves (grown in Wakayama prefecture) were subjected to a boil blanching treatment (95°C, 1 minute) with 1 L of hot boiled water, then cooled with water of room temperature, and further subjected to hot air drying (65°C, 21 hours) to afford 6.5 g of boiled dried leaves.

Then, 291 mg of the dried leaves were extracted with hot water (90°C, 60 min, bath ratio of 50-fold), filtered with a 0.45 µm PES filter, and then lyophilized to afford 29 mg of an extract powder of green perilla hot-water extract (solid yield of 10.1%). The rosmarinic acid content in the obtained green perilla extract powder was 25.3 mass%.

### Example 35

50.4 g of green perilla fresh leaves (grown in Wakayama prefecture) were subjected to a boil blanching treatment (95°C, 1 minute) with 1 L of 5% aqueous common salt solution, then cooled with water of room temperature, and further subjected to hot air drying (65°C, 21 hours) to afford 7.4 g of boiled dried leaves.

Then, 297 mg of the dried leaves were extracted with hot water (90°C, 60 min, bath ratio of 50-fold), filtered with a 0.45 µm PES filter, and then lyophilized to afford 59 mg of an extract powder of green perilla hot-water extract (solid yield of 19.8%). The rosmarinic acid content in the obtained green perilla extract powder was 20.8 mass%.

### Example 36

50.1 g of green perilla fresh leaves (grown in Wakayama prefecture) were subjected to a boil blanching treatment (95°C, 1 minute) with 1 L of 5% aqueous citric acid solution, then cooled with water of room temperature, and further subjected to hot air drying (65°C, 21 hours) to afford 7.0 g of boiled dried leaves.

Then, 279 mg of the dried leaves were extracted with hot water (90°C, 60 min, bath ratio of 50-fold), filtered with a 0.45 µm PES filter, and then lyophilized to afford 37 mg of an extract powder of green perilla hot-water extract (solid yield of 13.2%). The rosmarinic acid content in the obtained green perilla extract powder was 27.8 mass%.

### Comparative Example 7

50.6 g of green perilla fresh leaves (grown in Wakayama prefecture) were subjected to a boil blanching treatment (75°C, 1 minute) with 1 L of hot water, then cooled with water of room temperature, and further subjected to hot air drying (65°C, 21 hours) to afford 7.0 g of boiled dried leaves.

Then, 307 mg of the dried leaves were extracted with hot water (90°C, 60 min, bath ratio of 50-fold), filtered with a 0.45 µm PES filter, and then lyophilized to afford 46 mg of an extract powder of green perilla hot-water extract (solid yield of 15.1%). The rosmarinic acid content in the obtained green perilla extract powder was 5.2 mass%.

### Comparative Example 8

50.0 g of green perilla fresh leaves (grown in Wakayama prefecture) were subjected to a boil blanching treatment (60°C, 1 minute) with 1 L of hot water, then cooled with water of room temperature, and further subjected to hot air drying (65°C, 21 hours) to afford 7.0 g of boiled dried leaves.

Then, 296 mg of the dried leaves were extracted with hot water (90°C, 60 min, bath ratio of 50-fold), filtered with a 0.45 µm PES filter, and then lyophilized to afford 43 mg of an extract powder of green perilla hot-water extract (solid yield of 14.5%). The rosmarinic acid content in the obtained green perilla extract powder was 0.4 mass%.

### Example 37

59.6 g of spearmint fresh leaves (grown in Wakayama prefecture) were subjected to a boil blanching treatment (95°C, 1 minute) with 1 L of hot boiled water, then cooled with water of room temperature, and further subjected to hot air drying (65°C, 21 hours) to afford 6.4 g of boiled dried leaves.

Then, 287 mg of the dried leaves were extracted with hot water (90°C, 60 min, bath ratio of 50-fold), filtered with a 0.45 µm PES filter, and then lyophilized to afford 41 mg of an extract powder of spearmint hot-water extract (solid yield of 14.4%). The rosmarinic acid content in the obtained spearmint extract powder was 4.0 mass%.

### Example 38

52.1 g of spearmint fresh leaves (grown in Wakayama prefecture) were subjected to a boil blanching treatment (95°C, 1 minute) with 1 L of 5% aqueous common salt solution, then cooled with water of room temperature, and further subjected to hot air drying (65°C, 21 hours) to afford 8.8 g of boiled dried leaves.

Then, 281 mg of the dried leaves were extracted with hot water (90°C, 60 min, bath ratio of 50-fold), filtered with a 0.45 µm PES filter, and then lyophilized to afford 87 mg of an extract powder of spearmint hot-water extract (solid yield of 30.8%). The rosmarinic acid content in the obtained spearmint extract powder was 5.3 mass%.

### Example 39

58.6 g of spearmint fresh leaves (grown in Wakayama prefecture) were subjected to a boil blanching treatment (95°C, 1 minute) with 1 L of 5% aqueous citric acid solution, then cooled with water of room temperature, and further subjected to hot air drying (65°C, 21 hours) to afford 6.9 g of boiled dried leaves.

Then, 272 mg of the dried leaves were extracted with hot water (90°C, 60 min, bath ratio of 50-fold), filtered with a 0.45 µm PES filter, and then lyophilized to afford 68 mg of an extract powder of spearmint hot-water extract (solid yield of 25.1%). The rosmarinic acid content in the obtained spearmint extract powder was 5.1 mass%.

### Comparative Example 9

56.7 g of spearmint fresh leaves (grown in Wakayama prefecture) were subjected to a boil blanching treatment (75°C, 1 minute) with 1 L of hot water, then cooled with water of room temperature, and further subjected to hot air drying (65°C, 21 hours) to afford 6.4 g of boiled dried leaves.

Then, 289 mg of the dried leaves were extracted with hot water (90°C, 60 min, bath ratio of 50-fold), filtered with a 0.45 µm PES filter, and then lyophilized to afford 56 mg of an extract powder of spearmint hot-water extract (solid yield of 19.5%). The rosmarinic acid content in the obtained spearmint extract powder was 1.7 mass%.

### Comparative Example 10

59.4 g of spearmint fresh leaves (grown in Wakayama prefecture) were subjected to a boil blanching treatment (60°C, 1 minute) with 1 L of hot water, then cooled with water of room temperature, and further subjected to hot air drying (65°C, 21 hours) to afford 8.8 g of boiled dried leaves.

Then, 293 mg of the dried leaves were extracted with hot water (90°C, 60 min, bath ratio of 50-fold), filtered with a 0.45 µm PES filter, and then lyophilized to afford 68 mg of an extract powder of spearmint hot-water extract (solid yield of 23.10). The rosmarinic acid content in the obtained spearmint extract powder was 0.7 mass%.

### Example 40

56.5 g of rosemary fresh leaves (grown in Wakayama prefecture) were subjected to a boil blanching treatment (95°C, 1 minute) with 1 L of hot boiled water, then cooled with water of room temperature, and further subjected to hot air drying (65°C, 21 hours) to afford 14.6 g of boiled dried leaves.

Then, 290 mg of the dried leaves were extracted with hot water (90°C, 60 min, bath ratio of 50-fold), filtered with a 0.45 µm PES filter, and then lyophilized to afford 48 mg of an extract powder of rosemary hot-water extract (solid yield of 16.6%). The rosmarinic acid content in the obtained rosemary extract powder was 3.7 mass%.

### Example 41

54.5 g of rosemary fresh leaves (grown in Wakayama prefecture) were subjected to a boil blanching treatment (95°C, 1 minute) with 1 L of 5% aqueous citric acid solution, then cooled with water of room temperature, and further subjected to hot air drying (65°C, 21 hours) to afford 15.7 g of boiled dried leaves.

Then, 269 mg of the dried leaves were extracted with hot water (90°C, 60 min, bath ratio of 50-fold), filtered with a 0.45 µm PES filter, and then lyophilized to afford 58 mg of an extract powder of rosemary hot-water extract (solid yield of 21.5%). The rosmarinic acid content in the obtained rosemary extract powder was 3.5 mass%.

### Comparative Example 11

50.4 g of rosemary fresh leaves (grown in Wakayama prefecture) were subjected to a boil blanching treatment (60°C, 1 minute) with 1 L of hot water, then cooled with water of room temperature, and further subjected to hot air drying (65°C, 21 hours) to afford 13.7 g of boiled dried leaves.

Then, 303 mg of the dried leaves were extracted with hot water (90°C, 60 min, bath ratio of 50-fold), filtered with a 0.45 µm PES filter, and then lyophilized to afford 44 mg of an extract powder of rosemary hot-water extract (solid yield of 14.7%). The rosmarinic acid content in the obtained rosemary extract powder was 0.2 mass%.

The treatment conditions and results are shown in Table 9.

**[Table 9]**

| Example | Boiling conditions | | | | Hot-water extraction conditions | | | Hot-water extract yield | | Composition | | | Component amount ratio | |
|---|---|---|---|---|---|---|---|---|---|---|---|---|---|---|
| | Raw material leaves | Boiling water | Temperature °C | Time min | Temperature °C | Time min | Bath ratio fold | Solid yield % | RA recovered amount mg/1 g leaves | RA mass% | L7DG mass% | Lut mass ppm | RAIL7DG | RA/Lut |
| Example 34 | Green perilla leaves grown in Wakayama prefecture | Water | 90 | 1 | 90 | 60 | 50 | 10.1 | 25.5 | 25.3 | 0.6 | 0 | 24.7 | - |
| Example 35 | | 5 mass% aqueous common salt solution | 90 | 1 | 90 | 60 | 50 | 19.8 | 41.2 | 20.8 | 1.0 | 3 | 20.4 | 66221 |
| Example 36 | | 5 mass% aqueous citric acid solution | 90 | 1 | 90 | 60 | 50 | 13.2 | 36.7 | 27.8 | 0.6 | 37 | 28.8 | 4836 |
| Comparative Example 7 | | Water | 75 | 1 | 90 | 60 | 50 | 15.1 | 7.5 | 5.2 | 0 | 458 | - | 83 |
| Comparative Example 8 | | Water | 60 | 1 | 90 | 60 | 50 | 14.5 | 0.6 | 0.4 | 0.1 | 303 | 0.8 | 2 |
| Example 37 | Spearmint leaves grown in Wakayama prefecture | Water | 90 | 1 | 90 | 60 | 50 | 14.4 | 5.7 | 4.0 | 0 | 28 | - | 1270 |
| Example 38 | | 5 mass% aqueous common salt solution | 90 | 1 | 90 | 60 | 50 | 30.8 | 16.2 | 5.3 | 0 | 0 | - | - |
| Example 39 | | 5 mass% aqueous citric acid solution | 90 | 1 | 90 | 60 | 50 | 25.1 | 12.8 | 5.1 | 0 | 7 | - | 7439 |
| Comparative Example 9 | | Water | 75 | 1 | 90 | 60 | 50 | 19.5 | 3.3 | 1.7 | 0 | 25 | - | 706 |
| Comparative Example 10 | | Water | 60 | 1 | 90 | 60 | 50 | 23.1 | 1.7 | 0.7 | 0 | 20 | - | 389 |
| Example 40 | Rosemary leaves grown in Wakayama prefecture | Water | 90 | 1 | 90 | 60 | 50 | 16.6 | 0.6 | 3.7 | 0 | 37 | - | 1818 |
| Example 41 | | 5 mass% aqueous citric acid solution | 90 | 1 | 90 | 60 | 50 | 21.5 | 0.7 | 3.5 | 0 | 49 | - | 1412 |
| Comparative Example 11 | | Water | 60 | 1 | 90 | 60 | 50 | 14.7 | 0.3 | 0.2 | 0.4 | 0 | 10.4 | - |

### Example 42

57.1 g of green perilla fresh leaves (grown in Wakayama prefecture) were subjected to a steam treatment (98°C, 3 minutes) with boiling water, then cooled with water of room temperature, and further subjected to hot air drying (65°C, 21 hours) to afford 8.0 g of steamed dried leaves.

Then, 283 mg of the dried leaves were extracted with hot water (90°C, 60 min, bath ratio of 50-fold), filtered with a 0.45 µm PES filter, and then lyophilized to afford 39 mg of an extract powder of green perilla hot-water extract (solid yield of 13.9%). The rosmarinic acid content in the obtained green perilla extract powder was 15.2 mass%.

### Example 43

49.2 g of spearmint fresh leaves (grown in Wakayama prefecture) were subjected to a steam treatment (98°C, 3 minutes) with boiling water, then cooled with water of room temperature, and further subjected to hot air drying (65°C, 21 hours) to afford 7.8 g of steamed dried leaves.

Then, 276 mg of the dried leaves were extracted with hot water (90°C, 60 min, bath ratio of 50-fold), filtered with a 0.45 µm PES filter, and then lyophilized to afford 81 mg of an extract powder of green perilla hot-water extract (solid yield of 29.3%). The rosmarinic acid content in the obtained green perilla extract powder was 7.4 mass%.

### Example 44

53.7 g of rosemary fresh leaves (grown in Wakayama prefecture) were subjected to a steam treatment (98°C, 3 minutes) with boiling water, then cooled with water of room temperature, and further subjected to hot air drying (65°C, 21 hours) to afford 14.8 g of steamed dried leaves.

Then, 289 mg of the dried leaves were extracted with hot water (90°C, 60 min, bath ratio of 50-fold), filtered with a 0.45 µm PES filter, and then lyophilized to afford 56 mg of an extract powder of green perilla hot-water extract (solid yield of 19.4%). The rosmarinic acid content in the obtained green perilla extract powder was 7.1 mass%.

The treatment conditions and results are shown in Table 10.

**[Table 10]**

| | Blanching conditions | | | | Hot-water extraction conditions | | | Hot-water extract yield | | Composition |
|---|---|---|---|---|---|---|---|---|---|---|
| | Raw material leaves | Treatment method | Temperature °C | Time min | Temperature °C | Time min | Bath ratio fold | Solid yield % | RA recovered amount mg/1 g leaves | RA mass% |
| Example 42 | Green perilla leaves grown in Wakayama prefecture | Steaming | 90 | 3 | 90 | 60 | 50 | 13.9 | 21.1 | 15.2 |
| Example 43 | Spearmint leaves grown in Wakayama prefecture | Steaming | 90 | 3 | 90 | 60 | 50 | 29.3 | 21.8 | 7.4 |
| Example 44 | Rosemary leaves grown in Wakayama prefecture | Steaming | 90 | 3 | 90 | 60 | 50 | 19.4 | 13.9 | 7.1 |

## Claims

1. A method for producing a hot-water extract of a Lamiaceae plant, the method comprising the following steps (A) and (B):
(A) subjecting a Lamiaceae plant to a blanching treatment at 85°C or higher, then drying; and
(B) extracting a dried product obtained in the step (A) with hot water.

2. The method for producing a hot-water extract of a Lamiaceae plant according to claim 1, wherein the blanching treatment is a steam blanching treatment or a boil blanching treatment.

3. The method for producing a hot-water extract of a Lamiaceae plant according to claim 1 or 2, wherein the blanching treatment is performed in the presence of common salt or an organic acid.

4. The method for producing a hot-water extract of a Lamiaceae plant according to claim 3, wherein the organic acid is one or more selected from the group consisting of hydroxycarboxylic acids and ascorbic acids.

5. The method for producing a hot-water extract of a Lamiaceae plant according to claim 3, wherein the organic acid is citric acid, L-ascorbic acid, or a combination thereof.

6. The method for producing a hot-water extract of a Lamiaceae plant according to any one of claims 1 to 5, wherein a temperature of the hot water used for the extracting is from 40 to 100°C.

7. The method for producing a hot-water extract of a Lamiaceae plant according to any one of claims 1 to 6, wherein the Lamiaceae plant is one or more selected from the group consisting of plants of genus Perilla of family Lamiaceae, plants of genus Salvia of family Lamiaceae, and plants of genus Mentha of family Lamiaceae.

8. The method for producing a hot-water extract of a Lamiaceae plant according to any one of claims 1 to 6, wherein the Lamiaceae plant is one or more selected from the group consisting of red perilla, green perilla, rosemary, and spearmint.

9. The method for producing a hot-water extract of a Lamiaceae plant according to any one of claims 1 to 8, wherein the hot-water extract of a Lamiaceae plant is an extract having a mass ratio of a content of rosmarinic acid to a content of luteolin being 1,100 or more.

10. A hot-water extract of a Lamiaceae plant, having a mass ratio of a content of rosmarinic acid to a content of luteolin being 1,100 or more.

11. A method for producing a dried product of a Lamiaceae plant, the method comprising the following step (A'):
(A') subjecting a Lamiaceae plant to a blanching treatment in the presence of an organic acid, then drying.

12. The method for producing a dried product of a Lamiaceae plant according to claim 11, wherein the organic acid is one or more selected from the group consisting of hydroxycarboxylic acids and ascorbic acids.

13. The method for producing a dried product of a Lamiaceae plant according to claim 11, wherein the organic acid is citric acid, L-ascorbic acid, or a combination thereof.
